(19) Europäisches Patentamt / European Patent Office / Office européen des brevets

(11) **EP 2 780 049 B1**

(12) **FASCICULE DE BREVET EUROPEEN**

(45) Date de publication et mention de la délivrance du brevet:
**18.04.2018 Bulletin 2018/16**

(21) Numéro de dépôt: **12808855.6**

(22) Date de dépôt: **14.11.2012**

(51) Int Cl.:
*A61L 27/06* *(2006.01)*  *A61L 27/30* *(2006.01)*
*A61L 27/50* *(2006.01)*  *A61F 2/28* *(2006.01)*
*B05D 1/18* *(2006.01)*  *C25D 11/02* *(2006.01)*
*A61L 27/32* *(2006.01)*  *A61L 31/16* *(2006.01)*

(86) Numéro de dépôt international:
**PCT/FR2012/000457**

(87) Numéro de publication internationale:
**WO 2013/072576 (23.05.2013 Gazette 2013/21)**

(54) **PROCÈDE DE TRAITEMENT DE SURFACE DES IMPLANTS OSSEUX EN TITANE UTILISANT SUCCESSIVEMENT UN BAIN D'HYDROXYDE DE SODIUM ET UNE ANODISATION**

VERFAHREN ZUR OBERFLÄCHENBEHANDLUNG VON TITANKNOCHENIMPLANTATEN MIT EINEM NATRIUMHYDROXIDBAD UND NACHFOLGENDER ANODISIERUNG

METHOD FOR THE SURFACE TREATMENT OF TITANIUM BONE IMPLANTS USING, IN ORDER, A SODIUM HYDROXIDE BATH AND ANODIZATION

(84) Etats contractants désignés:
**AL AT BE BG CH CY CZ DE DK EE ES FI GB GR HR HU IE IS IT LI LT LU LV MC MK MT NL NO PL PT RO RS SE SI SK SM TR**

(30) Priorité: **14.11.2011 FR 1103460**

(43) Date de publication de la demande:
**24.09.2014 Bulletin 2014/39**

(73) Titulaire: **OBL (Société Anonyme)**
**92320 Chatillon (FR)**

(72) Inventeur: **BARBAS, Alexandre**
**41100 Vendôme (FR)**

(74) Mandataire: **EIP**
**EIP Europe LLP**
**Fairfax House**
**15 Fulwood Place**
**London WC1V 6HU (GB)**

(56) Documents cités:
**WO-A1-2009/044203**

- **LIU X ET AL: "Surface nano-functionalization of biomaterials", MATERIALS SCIENCE AND ENGINEERING R: REPORTS, ELSEVIER SEQUOIA S.A., LAUSANNE, CH, vol. 70, no. 3-6, 22 novembre 2010 (2010-11-22), pages 275-302, XP027537227, ISSN: 0927-796X [extrait le 2010-11-22]**
- **KAR A ET AL: "Electrodeposition of hydroxyapatite onto nanotubular TiO2 for implant applications", SURFACE AND COATINGS TECHNOLOGY, ELSEVIER, AMSTERDAM, NL, vol. 201, no. 6, 4 décembre 2006 (2006-12-04), pages 3723-3731, XP024996420, ISSN: 0257-8972, DOI: 10.1016/J.SURFCOAT.2006.09.008 [extrait le 2006-12-04]**
- **KUKOBU: "Formation of biologically active bone-like apatite on metals and polymers by a biomimetic process", THERMOCHIMICA ACTA, vol. 280/281, 1996, pages 479-490, XP002692797,**

**Description**

**DOMAINE TECHNIQUE DE L'INVENTION**

La présente invention a trait à un procédé de traitement de surface d'un implant en titane bioinerte destiné à être posé en contact avec un os du corps humain, ledit procédé visant à rendre bioactif le titane bioinerte afin de permettre une liaison chimique de l'os avec l'implant.

Un tel traitement va donc favoriser l'ostéointégration de l'implant ainsi traité en surface, la liaison entre ledit l'implant et l'os étant, à la suite de ce traitement, de meilleure qualité et obtenue plus rapidement qu'avec un implant en titane classique. Il est dès lors raisonnablement possible d'espérer une augmentation significative de la durée de vie clinique de la solution implantaire.

**ARRIÈRE PLAN TECHNOLOGIQUE DE L'INVENTION**

La littérature nous a enseigné que les matériaux utilisés à des fins d'implantation dans le corps humain peuvent être de différentes natures.

En effet, il existe des matériaux biotolérants, des matériaux bioinertes et des matériaux bioactifs dont quelques exemples apparaissent sur le Tableau 1 établi par Kienapfel H., Sprey C., Wilke A., Griss P., Implant fixation by bone ingrowth, The Journal of Arthroplasty, Volume 14 n°3, 1999, lequel Tableau 1 détaille également le niveau de biocompatibilité de certains des matériaux examinés en contact avec l'os. La nuance entre ces types de matériaux se fait par la façon dont ils vont être acceptés par l'organisme. Ainsi, les matériaux biotolérants vont être encapsulés par un tissu fibreux. Les matériaux bioinertes ne vont pas engendrer de réaction des tissus vivants. L'os va pouvoir croître sur ces matériaux sans lien chimique. A l'inverse, les matériaux bioactifs vont s'intégrer parfaitement au milieu vivant en créant des liens chimiques avec celui-ci.

**Tableau 1**

| Matériaux | Etendue de la biocompatibilité | Ostéogenèse |
|---|---|---|
| PMMA (Polyméthacrylate de méthyle) | Biotolérant | Ostéogenèse à distance |
| Acier inoxydable | Biotolérant | Ostéogenèse à distance |
| Alumine | Bioinerte | Ostéogenèse de contact |
| Carbone | Bioinerte | Ostéogenèse de contact |
| Titane et alliages à base de titane | Bioinerte | Ostéogenèse de contact |
| Alliages chrome-cobalt | Bioinerte | Ostéogenèse avec liaison |
| Céramiques phosphocalciques | Bioactif | Ostéogenèse avec liaison |

Kokubo T. (voir « Bioactive glass ceramics : properties and applications, Biomaterials 12, pages 155-163, 1991 » et voir également « Formation of biologically active bone-like apatite on metals and polymers by a biomimetic process, Thermochimica Acta 280/281, pages 479-490, 1996 ») a effectué des recherches concluant au fait que la liaison entre le titane et l'os s'effectue par l'intermédiaire d'un film d'apatite, notamment sous la forme d'hydroxyapatite qui est une espèce minérale de la famille des phosphates, de formule $Ca^5(PO_4)^3(OH)$. On sait que le titane est bioinerte. L'os ne peut donc pas se lier chimiquement à ce dernier. Cependant, il a été observé que, à la suite d'une implantation en titane, les fluides biologiques déposent sur l'implant un film de phosphate de calcium qui va se lier chimiquement avec le titane de l'implant. Et c'est à ce dépôt, dans lequel les taux de calcium et de phosphore sont alors dans le rapport Ca/P voisin de 1,57 à 1,62, dont la constitution est donc très proche de celle de l'os humain, que l'os peut se lier chimiquement.

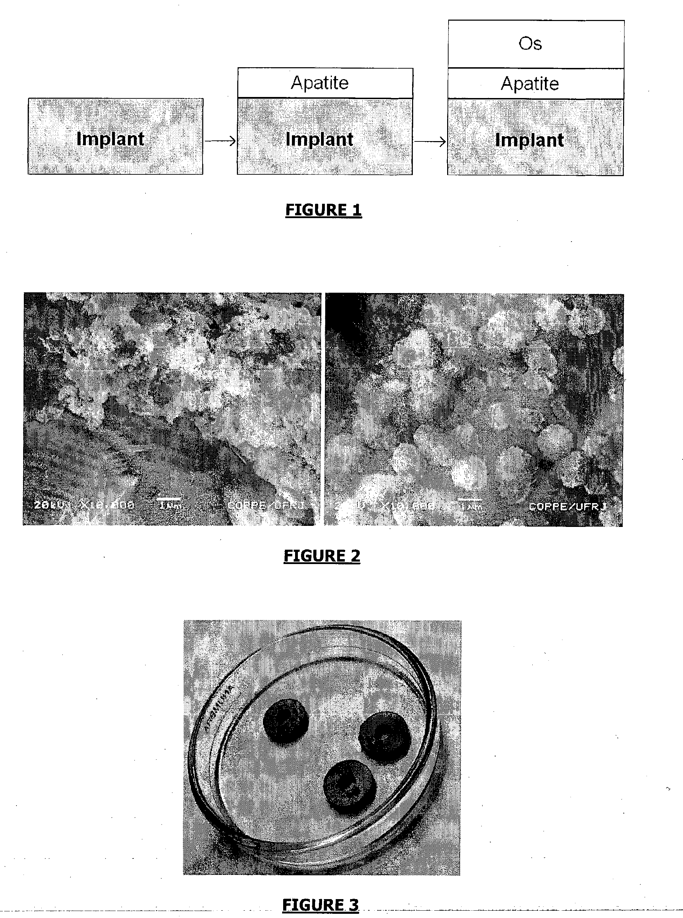

La Figure 1 schématise d'ailleurs le processus d'accroche de l'os sur un implant in vivo.

Des scientifiques ont à cette suite mis au point des techniques d'évaluation de la bioactivité en utilisant à cette fin des solutions qui simulent la partie minérale du plasma sanguin humain, qui contiennent donc les mêmes ions que le plasma sanguin, et à des concentrations équivalentes. Plusieurs solutions de ce type, dites « Simulated Body Fluid » ou encore SBF, ont vu le jour durant ces dernières années. Après le premier SBF créé par Kokubo en 1991, des améliorations ont en effet été apportées à ce fluide afin qu'il représente au mieux la partie minérale du plasma sanguin humain. Les propriétés chimiques du SBF lui permettent ainsi d'évaluer la bioactivité d'un matériau en immergeant ce dernier dans ledit fluide SBF pendant un certain temps et en observant sa surface après séchage.

Le Tableau 2, communiqué par Kokubo T., Takadama H. (How useful is SBF in predicting in vivo bone bioactivity, Biomaterials 27, pages 2907-2915, 2006), établit d'ailleurs la comparaison entre la composition du plasma sanguin humain et différents SBF.

**Tableau 2**

| | Concentration ionique (mM) | | | | | | | |
|---|---|---|---|---|---|---|---|---|
| | Na$^+$ | K$^+$ | Mg$^{2+}$ | Ca$^{2+}$ | Cl$^-$ | HCO$_3^-$ | HPO$_4^{2-}$ | SO$_4^{2-}$ |
| Plasma sanguin humain | 142,0 | 5,0 | 1,5 | 2,5 | 103,0 | 27,0 | 1,0 | 0,5 |
| SBF d'origine | 142,0 | 5,0 | 1,5 | 2,5 | 148,8 | 4,2 | 1,0 | 0 |
| SBF corrigé (c-SBF) | 142,0 | 5,0 | 1,5 | 2,5 | 147,8 | 4,2 | 1,0 | 0,5 |
| SBF revisé (r-SBF) | 142,0 | 5,0 | 1,5 | 2,5 | 103,0 | 27,0 | 1,0 | 0,5 |
| SBF amélioré résultant | 142,0 | 5,0 | 1,5 | 2,5 | 103,0 | 4,2 | 1,0 | 0,5 |

D'après Kokubo, la couche indispensable à la liaison entre l'os et le titane *in vivo* peut être reproduite in vitro par immersion dans le SBF. La capacité d'un matériau à créer des liens avec l'os correspond à sa capacité à être recouvert d'une couche d'apatite lors de son immersion dans ce fluide corporel simulé *in vitro.* A l'inverse, la colonisation osseuse ne pourra pas se faire sur un matériau qui ne présente pas de dépôt d'apatite après son immersion dans le SBF.

L'observation du dépôt d'apatite peut se faire par microscopie électronique, et l'analyse de la composition de ce dépôt peut être effectuée par tout procédé d'analyse chimique.

Toujours selon Kokubo, il est en conséquence possible de comparer la bioactivité de plusieurs matériaux en comparant la vitesse de déposition de l'apatite sur ces différents matériaux lors de leur immersion dans du SBF.

En 2008, s'intéressant plus particulièrement au titane pur, l'équipe de Waléria Silva de Meideros, de Oliveira M.V., Pereira L.C. et de Andrade M.C. (Bioactive Porous Titanium : an alternative to surgical implants, Artificial Organs 32(4), pages 277-282, 2008) a immergé dans du SBF des échantillons poreux en titane pur. Cette équipe a conclu que, après sept jours, un dépôt de calcium était identifiable. Puis, à partir de quatorze jours, il lui a été possible d'observer par analyse EDX la présence de calcium et de phosphore. D'ailleurs, comme le montre la Figure 2, on peut constater qu'après quatorze et, respectivement, vingt-huit jours d'immersion, un film de phosphate de calcium est présent en surface du matériau.

Selon Kokubo, dans son article précité paru en 1996 dans « Thermochimica Acta », la couche d'apatite qui se dépose sur le matériau implanté dans le corps humain se fait grâce aux groupes hydroxyles (OH) présents en surface dudit matériau. Ces groupes hydroxyles apparaissent grâce à l'action des fluides biologiques sur le matériau. Une couche contenant des oxydes de titane hydratés (HTiO$_3^-$) peut être reproduite, pour le titane, en l'immergeant dans une solution contenant de l'hydroxyde de sodium NaOH concentré à 10 moles par litre. En effet, l'immersion du titane dans cette solution permet de dissoudre la couche d'oxyde passive en surface et de créer une nouvelle couche d'oxydes nécessaires à l'accroche de la couche d'apatite. Le même auteur nous informe sur le fait que cette nouvelle couche d'oxydes est instable à la fois mécaniquement et chimiquement. Pour la stabiliser, il réalise un traitement thermique à 600°C afin de rendre ce dépôt amorphe et cristallin. La présence de cette couche facilite l'apparition, en surface du matériau, des groupes hydroxyles (TiOH) qui vont induire la formation d'apatite.

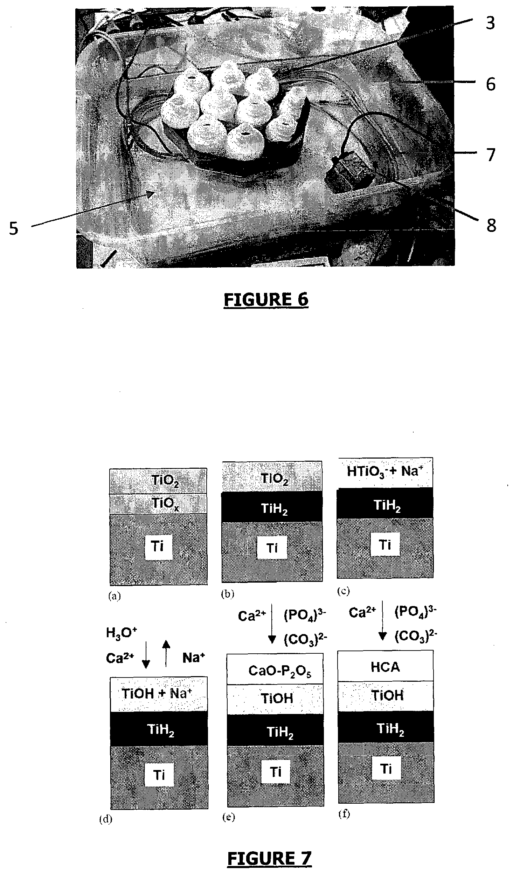

Lenka Jonasova et son équipe (Jonasova L., Müller F.A., Helebrant A., Strnad J., Greil P., Biomimetic apatite formation on chemically treated titanium, Biomaterials 25, pages 1187-1194, 2004) ont travaillé sur ce même sujet. Mais, pour leur part, ils ont cherché à améliorer un tel traitement du titane à l'hydroxyde de sodium en préparant préalablement le titane et, plus précisément, en le décapant avec une solution contenant du HCl. D'après leurs travaux, cette préparation préalable permet d'obtenir, avant le traitement au NaOH, une couche de TiO2 plus fine et plus uniforme que sans un tel décapage. Des échantillons qui avaient été traités avec ce procédé (HCl, puis NaOH) ont ensuite été immergés dans du SBF. A la suite de chaque étape, des analyses chimiques ont été effectuées et des suppositions sur les réactions chimiques ayant eu lieu, conduisant à des modifications de la surface des échantillons de titane, ont été émises comme on peut le voir sur la Figure 7.

Il semble que le décapage parvienne à dégrader la couche de TiO2 présente naturellement en surface du titane et que, à la suite de cette dégradation, il se forme une couche de TiH2. Au contact de l'air ambiant, une nouvelle couche de TiO2 plus fine se forme. L'immersion dans l'hydroxyde de sodium NaOH permet de dissoudre cette couche superficielle de TiO2 et de former un nouvel oxyde de titane contenant des ions Na$^+$. Lors de l'immersion dans le SBF, un échange d'ions se produit entre celui-ci et la surface du titane. Ceci conduit à la formation d'une couche de TiOH. Les ions Ca$^{2+}$ sont ensuite incorporés à cette couche. Et ce sont ces ions Ca$^{2+}$ qui, de par leur charge positive, vont permettre de former de l'apatite sur la surface. En effet, les ions (PO$_4$)$^{3-}$ et (CO$_3$)$^{2-}$ vont pouvoir s'y accrocher et ainsi former de l'apatite. L'apatite ici formée en surface est de l'HydroxyCarbonated Apatite (HCA), c'est-à-dire une hydroxyapatite proche de celle présente naturellement dans un tissu osseux.

LIU X ET AL: "Surface nano-functionalization of biomaterials", MATERIALS SCIENCE AND ENGINEERING R: REPORTS, ELSEVIER SEQUOIA S.A., LAUSANNE, CH, vol. 70, no. 3-6, 22 novembre 2010 (2010-11-22), pages 275-302, XP027537227, ISSN: 0927-796X, étudie la nano-fonctionnalisation de surface de biomatériaux métalliques comme le titane, par un traitement chimique (NaOH) ou par oxydation anodique. Puisque ce document ne décrit pas ces deux techniques combinées.

KAR A ET AL: "Electrodeposition of hydroxyapatite onto nanotubular Ti02 for implant applications", SURFACE AND COATINGS TECHNOLOGY, ELSEVIER, AMSTERDAM, NL, vol. 201, no. 6, 4 décembre 2006 (2006-12-04), pages 3723-3731, XP024996420, ISSN: 0257-8972, DOi: 10.1016/J.SURFCOAT.2006.09.008, décrit une méthode de traitement de la surface d'implants en titane comprenant anodisation de la surface, le traitement chimique de la surface anodisée dans une solution de NaOH à une concentration de 10 moles par litres et le dépôt d'un revêtement d'hydroxyapatite. Puisque ce document ne décrit pas d'abord le traitement chimique et ensuite l'anodisation. Cela étant, il n'a été tiré aucune conclusion de toutes ces hypothèses formulées dans la littérature alors pourtant que les implants en titane sont de plus en plus utilisés et que sont même apparus récemment des implants en titane poreux dont les caractéristiques de porosité sont très voisines de celles de l'os humain.

Aux termes d'une demande de brevet déposée en parallèle de celle-ci par la même société déposante, il a été proposé un procédé nouveau de traitement de surface d'un implant en titane par immersion dans un bain d'hydroxyde de sodium qui modifie la couche d'oxydes présente naturellement sur le titane et qui rend bioactif ce métal naturellement bioinerte, qui permet donc une forte liaison chimique de l'os avec l'implant réalisé en un tel métal et qui, par voie de conséquence, favorise l'ostéointragration dudit implant.

La stabilité chimique et mécanique des effets de ce traitement à l'hydroxyde de sodium n'est toutefois pas certaine.

La présente invention se propose donc de pallier cet éventuel inconvénient.

## DESCRIPTION GÉNÉRALE DE L'INVENTION

La présente invention a donc pour premier objet un procédé de traitement de surface d'un implant en titane bioinerte destiné à être posé en contact avec un os du corps humain, du type consistant à laisser des fluides biologiques, tels que la partie minérale du plasma sanguin humain, déposer naturellement sur l'implant en titane un film de phosphate de calcium, de formule $Ca^5(PO_4)^3(OH)$, sous forme d'hydroxyapatite, en vue de rendre bioactif le titane bioinerte afin de permettre une liaison chimique de l'os avec l'implant, favorisant ainsi l'ostéointégration, le procédé comprenant une opération consistant à immerger le titane dans une solution contenant de l'hydroxyde de sodium NaOH dont la concentration est de l'ordre de 10 moles par litre, ladite immersion entraînant la formation sur le titane d'une couche d'oxydes de titane hydratés $(HTiO_3-)$ qui entraîne elle-même l'apparition de groupes hydroxyles TiOH permettant le dépôt dudit film de phosphate de calcium, caractérisé en ce qu'il comprend, après l'opération d'immersion de l'implant dans l'hydroxyde de sodium, une opération d'anodisation de l'implant sous une tension donnée permettant une stabilisation de la couche d'oxydes formée par l'opération d'immersion dans l'hydroxyde de sodium.

La tension de l'opération d'anodisation est avantageusement de l'ordre de 50 à 110 volts.

De préférence, la tension de l'opération d'anodisation est une faible tension, et elle est de préférence encore de l'ordre de 50 à 75 volts.

Dans un mode opératoire particulièrement avantageux, la tension de l'opération d'anodisation est de l'ordre de 50 volts.

Selon une variante, la tension de l'opération d'anodisation est de l'ordre de 110 volts.

Selon une autre particularité intéressante, chacune des opérations d'immersion dans l'hydroxyde de sodium et d'anodisation dudit procédé est suivie d'une opération de rinçage.

L'ensemble des deux opérations d'immersion dans l'hydroxyde de sodium et d'anodisation pourra être avantageusement suivi d'une opération de séchage, et dans un tel cas l'opération de séchage est effectuée à une température de l'ordre de 100°C.

Selon encore une dernière particularité, l'ensemble desdites opérations indiquées est suivi d'un nettoyage-lavage standard.

La présente invention a bien évidemment pour second objet tout implant en titane obtenu par la mise en oeuvre d'un procédé répondant aux caractéristiques précitées.

Les spécifications détaillées de l'invention sont données dans la description qui suit en liaison avec les dessins ci-annexés. Il est à noter que ces dessins n'ont d'autre but que celui d'illustrer le texte de la description et qu'ils ne constituent donc en aucune sorte une limitation de la portée de l'invention.

## BRÈVE DESCRIPTION DES DESSINS

La Figure 1 est un schéma du processus d'accroche de l'os sur les implants irrigués par les fluides corporels.

La Figure 2 reproduit les observations au microscope électronique à balayage d'un dépôt de phosphate de calcium sur des échantillons en titane pur après 14 (à gauche) et 28 (à droite) jours d'immersion dans du SBF.

La Figure 3 montre un exemple de réalisation d'échantillons en forme de pastille trouée, utilisés pour les essais de bioactivité.

La Figure 4 représente un porte-échantillons destiné à porter les pastilles de la Figure 3, ledit porte-échantillons étant lui-même fixé au bouchon d'une bouteille utilisée pour les essais de bioactivité.

La Figure 5 représente en coupe verticale le schéma d'une bouteille utilisée aux fins précitées, ladite bouteille contenant en conséquence le porte-échantillons et du SBF.

La Figure 6 est une vue du dispositif expérimental utilisé.

La Figure 7 détaille les modifications, selon Lenka Jonasova et son équipe, de la surface du titane : pendant le décapage au HCl (a-b), pendant le traitement au NaOH (b-c) et durant les phases de dépôt d'apatite dans le SBF (d-f).

La Figure 8 est une comparaison des zones observées en microscopie électronique à balayage des échantillons, respectivement A, B, C et F de gauche à droite puis de haut en bas, après leur immersion dans le SBF.

La Figure 9 est une comparaison des zones observées en microscopie électronique à balayage des échantillons D (à gauche) et E (à droite) après leur immersion dans le SBF.

## DESCRIPTION DES MODES DE RÉALISATION PRÉFÉRÉS DE L'INVENTION

En préliminaire de la description détaillée de son invention, la société déposante juge nécessaire de rappeler que, dans des domaines voisins de celui directement visé par son invention, il est connu d'utiliser des techniques dont la mise en oeuvre comprend des opérations dont certaines présentent quelques similitudes avec certaines des opérations effectuées au cours du procédé conforme à l'invention. Ces techniques voisines ne sauraient toutefois affecter la nouveauté ni l'originalité de la présente invention, et ne sauraient pas davantage rendre celle-ci évidente, les buts recherchés par ces différentes techniques étant en effet fort éloignés et les opérations effectuées tant avant qu'après celles présentant quelques similitudes entre elles étant, elles aussi, fort éloignées les unes des autres.

Il en est notamment ainsi du procédé décrit dans la demande de brevet internationale objet de la publication WO2009/044203 qui consiste à réaliser une anodisation de métaux pour ensuite y intégrer des éléments biocides. Le matériau biocide utilisé constamment dans le cadre de cette étude est l'argent.

Ce procédé comprend en effet successivement les étapes de nettoyage par ultrasons d'un implant métallique (par exemple en titane) plongé dans un bain d'acétone, suivi d'un rinçage à l'eau déionisée, puis d'un nettoyage dans une solution alcaline d'hydroxyde de sodium NaOH à 1 M, puis d'un nouveau rinçage à l'eau déionisée et enfin d'une anodisation entre 50 V et 150 V dans une solution d'acide phosphorique dont la concentration est de préférence comprise en 1 et 3 M.

L'anodisation est ensuite poursuivie en utilisant une tension qui est rendue négative, cette tension étant de l'ordre de -0,2 V à -0,7 V, et les ultimes phases de traitement consistant, après ces deux opérations d'anodisation, en un troisième rinçage à l'eau déionisée suivi d'une immersion dans une solution contenant un matériau biocide, en l'occurrence l'argent comme il a été dit en préambule.

Si ce procédé connu et le procédé de la présente invention ont en commun des opérations de nettoyage/décapage, de rinçage, d'immersion dans une solution d'hydroxyde de sodium et d'anodisation, en vue d'améliorer les caractéristiques de surface de certains implants métalliques, et notamment d'implants en titane, il en est fini de l'exposé de leurs points communs.

Ainsi, la phase commune d'immersion dans une solution alcaline d'hydroxyde de sodium est une phase de nettoyage de la surface de l'implant dans le procédé de l'art antérieur, réalisée avec une solution de NaOH concentrée à 1 M, alors que, dans le procédé conforme à l'invention, l'immersion dans la solution d'hydroxyde de sodium doit avoir pour conséquence de modifier la composition chimique de la surface de l'implant et elle est donc réalisée avec une solution de NaOH concentrée à 10 M.

Ainsi, également, la phase d'anodisation du procédé de l'art antérieur a pour but, du fait de sa particularité consistant à inverser la tension, d'assurer un traitement de surface de l'implant, en créant des porosités, alors que, dans le procédé conforme à l'invention, la phase d'anodisation a pour but de stabiliser mécaniquement et chimiquement la couche d'oxydes formée à la surface de l'implant, suite à l'immersion dudit implant dans la solution d'hydroxyde de sodium concentrée à 10 M. Or, pour mémoire, cette couche d'oxydes est inexistante dans le procédé de l'art antérieur dès lors que la phase d'immersion de l'implant dans l'hydroxyde de sodium permet seulement un simple nettoyage de la surface de l'implant car la concentration de 1 M est trop faible pour modifier la composition de ladite surface.

Ainsi enfin, quant aux buts, le procédé de l'art antérieur vise à créer en surface de l'implant des porosités au sein desquelles est placé un matériau biocide alors que le procédé conforme à l'invention est destiné à améliorer la bioactivité des implants en modifiant la couche d'oxydes présente en surface et en créant des liens chimiques forts entre cette couche et le milieu vivant, qui peut ainsi la coloniser plus rapidement et plus solidement.

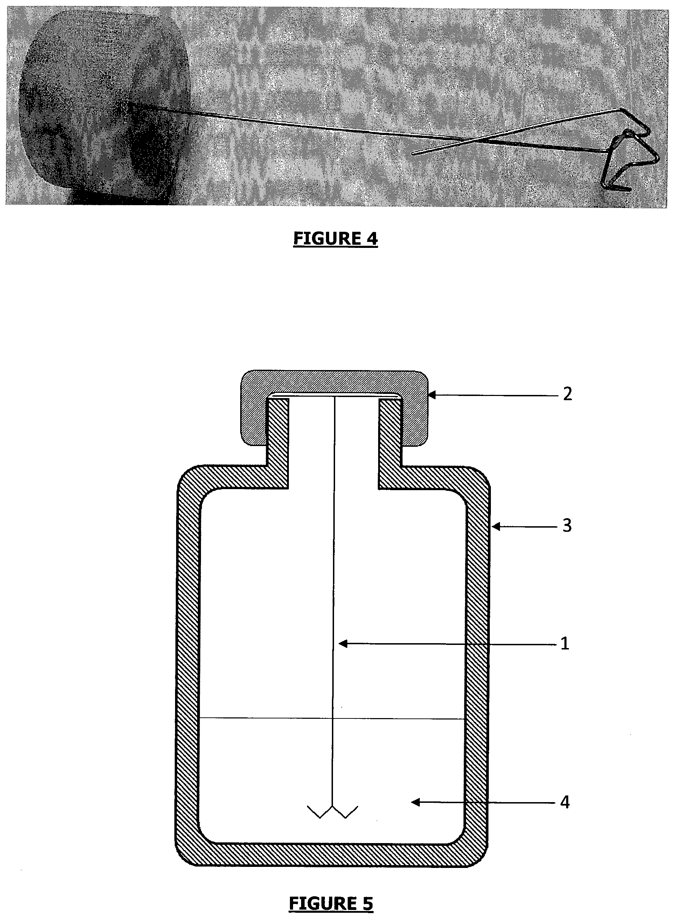

Ces différences ayant été rappelées, conformément à l'invention, un dispositif spécifique a été mis au point, un tel dispositif permettant de réaliser des essais d'immersion dans du SBF pour des échantillons ayant subi au préalable différents traitements de surface. Ce dispositif et ses différents accessoires sont représentés aux Figures 3 à 6. Les expériences menées ont bien sûr toutes en commun le fait de comprendre au final l'immersion d'échantillons en titane dans du SBF pendant un temps défini. Des échantillons spécifiques en forme de petites pastilles ont été conçus comme le montre la Figure 3. Ces pastilles, réalisées en titane de grade 2, ont un diamètre de 10 mm et une épaisseur de 1,5 mm. Le trou percé au centre desdites pastilles de titane permet de suspendre trois de ces pastilles sur un porte-échantillons 1 réalisé en fil d'acier inoxydable visible sur la Figure 4. Le fil est fixé au centre du bouchon 2 d'une bouteille 3 contenant du SBF 4, ce qui permet d'immerger les échantillons par groupe de trois dans le SBF. La Figure 5 schématise ce principe.

Afin d'être représentatives des phénomènes se déroulant dans le corps humain, les expériences finales d'immersion précitées sont réalisées à une température de 37°C. Pour cela, les bouteilles 3 sont immergées dans un bain-marie 5 dont la température est régulée à 37°C. La Figure 6 est une photographie du dispositif expérimental. Des bouteilles 3 contenant chacune trois échantillons de titane qui ont préalablement subi le même traitement de surface et qui baignent dans du SBF 4 sont suspendues au couvercle (non représenté) du bac principal 6, et sont mises en mouvement par une pompe d'agitation 8. Ladite pompe 8 permet à la fois d'homogénéiser la température de l'eau servant au bain-marie, et d'agiter les bouteilles contenant les échantillons de titane et le SBF.

Le procédé selon la présente invention propose d'adjoindre une opération d'anodisation à l'opération d'immersion dans l'hydroxyde de sodium décrite dans une demande de brevet déposée en parallèle de celle-ci, et ce afin de pallier une éventuelle instabilité chimique et mécanique des effets du traitement à l'hydroxyde de sodium. A ce propos, la déposante de la présente demande de brevet suspecte que la réalisation d'une anodisation après le traitement à l'hydroxyde de sodium précédemment décrit pourrait en effet permettre de stabiliser les oxydes présents à la surface du titane.

Afin d'évaluer et d'optimiser cet autre traitement, un plan d'expériences préliminaires de traitements de surface et d'anodisations a été établi. Le Tableau 3 présente les cinq types d'échantillons qui ont été fabriqués afin de connaître l'influence du traitement de surface sur leur bioactivité. Les traitements suivants ont été réalisés :

- traitement à l'hydroxyde de sodium à 10 M couplé à l'anodisation effectuée avant ou après ledit traitement, et ce pour essayer de stabiliser dans le temps, chimiquement et mécaniquement, la couche d'oxyde engendrée par le traitement à l'hydroxyde de sodium,
- anodisation à différentes tensions, avant ou après le traitement à l'hydroxyde de sodium.

Chaque type d'échantillon est triplé afin de s'assurer de la répétabilité des opérations effectuées, définies dans le Tableau 3 qui suit.

Pour toutes ces opérations de traitement de surface réalisées par immersion dans une solution d'hydroxyde de sodium, il a été effectué au préalable une opération de décapage à l'aide d'une solution contenant de l'acide fluoro-nitrique $HFNO_3$. Un tel décapage aurait toutefois pu aussi être effectué à l'aide d'une solution d'acide chlorhydrique.

**Tableau 3 : Différents traitements appliqués aux échantillons de titane**

| A | B | C |
|---|---|---|
| Décapage HFNO3, 1min | Décapage HFN03, 1min | Décapage HFNO3, 1min |
| Rinçages | Rinçages | Rinçages |
| NaOH 10M, 60°, 24h | NaOH 10M, 60°, 24h | NaOH 10M, 60°, 24h |
| Rinçage | Rinçage | Rinçage |
| Anodisation 50V | Anodisation 75V | Anodisation 110V |
| Rinçage + Séchage 100° | Rinçage + Séchage 100° | Rinçage + Séchage 100° |
| Nettoyage lavage standard | Nettoyage lavage standard | Nettoyage lavage standard |
| D | E | F |
| Décapage HFNO3, 1min | Décapage HFNO3, 1min | Décapage HFNO3, 1min |

(suite)

|  | D | E | F |
|---|---|---|---|
|  | Rinçages | Rinçages | Rinçages |
|  | Anodisation 90V | Anodisation 15V |  |
|  | Rinçages | Rinçages |  |
|  | NaOH 10M, 60°, 24h | NaOH 10M, 60°, 24h | NaOH 10M, 60°, 24h |
|  | Rinçage + Séchage 100° | Rinçage + Séchage 100° | Rinçage + Séchage 100°C |
|  | Nettoyage lavage standard | Nettoyage lavage standard | Nettoyage lavage standard |

**Analyse des résultats**

Après avoir reçu de tels traitements de surface et d'anodisations, les différents échantillons ainsi traités sont immergés pendant une semaine dans le SBF (r-SBF, voir Tableau 2), puis sont observés par microscopie électronique. Des analyses chimiques sont réalisées afin de connaître la composition de l'apatite formée en surface. Trois analyses chimiques sur des zones de $240{\times}240\mu m^2$ sont réalisées sur chaque échantillon afin d'améliorer les statistiques. Il est rappelé que les principaux composants de l'apatite sont le calcium et le phosphore. Leur quantité respective définit la « qualité » du dépôt formé. En effet, un rapport Ca/P compris entre 0,5 et 2 est nécessaire pour s'assurer que le dépôt est bien de l'apatite (Driessens F.C.M., Boltong M.G., de Maeyer E.A.P., Wenz R., Nies B., Planell J.A., The Ca/P range of nanoapatitic calcium phosphate cements, Biomaterials 23, pages 4011-4017, 2002). Enfin, l'on sait également que, plus les quantités de calcium et de phosphore sont importantes, plus le traitement est efficace.

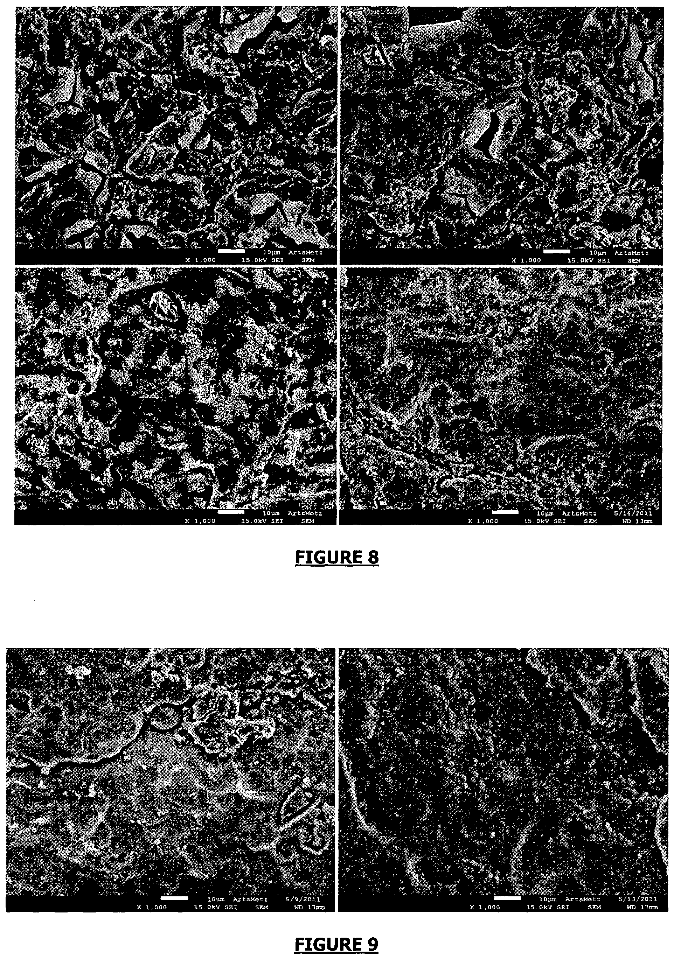

En comparant les données du Tableau 4 pour les échantillons A, B et C, on remarque que la tension d'anodisation a une influence sur le dépôt qui s'effectue lors de l'immersion. En effet, une faible tension permet un dépôt de calcium et de phosphore important. L'augmentation de la tension fait chuter les taux de ces éléments ainsi que le rapport Ca/P. Le traitement NaOH + anodisation est intéressant car, si on compare les traitements (A, B, C) à un traitement à l'hydroxyde de sodium uniquement (échantillon F), on remarque que le rapport Ca/P est bien plus faible (inférieur à 2 pour NaOH + anodisation contre 4,3 pour le traitement NaOH seul) comme l'indique le Tableau 4. Le composé formé à la surface des échantillons ayant subi les deux traitements consécutifs est donc bien de l'apatite. De plus, on peut remarquer que les taux de calcium et de phosphore sont bien plus importants pour le traitement combiné à faible tension d'anodisation que pour le traitement à l'hydroxyde de sodium seul. En observant la Figure 8, on remarque que la tension d'anodisation a une grande influence sur l'état de surface des échantillons. En effet, à faible tension, la couche provenant du traitement à l'hydroxyde de sodium est altérée mais on retrouve les écailles caractéristiques d'une surface traitée à l'hydroxyde de sodium comme le montre l'image de l'échantillon F. A tension moyenne, les écailles sont plus grosses mais toujours présentes. Par contre pour une tension de 110V, la couche du traitement à l'hydroxyde de sodium paraît avoir été totalement modifiée ; on remarque sur la Figure 8 une surface oxydée. Ceci conduit à un rapport Ca/P de 1,22, rapport qui est convenable, mais toutefois les quantités de calcium et de phosphore sont plus faibles que pour les échantillons A et B.

Les échantillons D et E ont subi un traitement combiné inversé : anodisation puis NaOH. Il est apparu en effet souhaitable de déterminer l'influence de la préparation de la surface des échantillons par une anodisation contrôlée avant d'effectuer le traitement à l'hydroxyde de sodium à 10 M. Contrairement aux échantillons A, B et C, l'anodisation a donc eu lieu avant le traitement à NaOH. Après immersion, une anodisation à faible voltage (échantillon E) permet d'augmenter les taux de calcium et de phosphore tout en faisant chuter le rapport Ca/P par rapport au traitement NaOH seul. L'anodisation à tension élevée (échantillon D) engendre un dépôt de phosphore équivalent au traitement à NaOH seul, mais un dépôt de calcium moins important. Ceci engendre un rapport Ca/P beaucoup plus faible.

Le traitement par anodisation suivie d'une immersion dans NaOH est donc moins efficace que le traitement NaOH suivi d'une anodisation. En effet, il engendre un dépôt de calcium et de phosphore plus faible en quantité et un rapport Ca/P plus élevé. Le traitement à l'hydroxyde de sodium a pour effet de détruire la couche de $TiO_2$ afin de la remplacer.par un oxyde de titane hydraté. Or l'anodisation réalisée avant le traitement à l'hydroxyde de sodium fait croître l'épaisseur de la couche de $TiO_2$ présente initialement à la surface du titane. Ceci rend le processus de désoxydation par l'hydroxyde de sodium beaucoup plus difficile. Ainsi, l'effet du traitement à l'hydroxyde de sodium est réduit.

En comparant ces résultats aux analyses faites sur l'échantillon F, d'après le Tableau 4, on remarque que le dépôt sur cet échantillon possède le rapport Ca/P le plus élevé. Le dépôt est donc de moins bonne qualité que ceux présents sur les autres échantillons. De plus, on constate que les taux de calcium et de phosphore sur l'échantillon F ne sont pas très élevés. En effet, les échantillons A et B présentent une quantité de calcium et de phosphore bien plus importante pour un rapport Ca/P intéressant.

**Tableau 4 : Moyenne du taux de Ca et P présents en surface des échantillons et rapport Ca/P correspondant, après leur immersion dans le SBF**

|   | Ca | P | Ca/P |
|---|---|---|---|
| A | 1,45±0.03 | 0,86±0.05 | 1,68±0.06 |
| B | 1,21±0.05 | 0,58±0.04 | 2,07±0.08 |
| C | 0,47±0.09 | 0,39±0.07 | 1,22±0.18 |
| D | 0,62±0.11 | 0,23±0.05 | 2,73±0.23 |
| E | 1,22±0.16 | 0,39±0.07 | 3,10±0.20 |
| F | 0,99±0.05 | 0,23±0.04 | 4,29±0.59 |

**Bilan**

L'invention propose un nouveau traitement de surface basé sur le traitement à l'hydroxyde de sodium à une concentration de l'ordre de 10 M suivi d'une anodisation. L'anodisation doit stabiliser la couche d'oxydes formée par le traitement à l'hydroxyde de sodium. Il a en outre été montré que le traitement consistant en une immersion dans de l'hydroxyde de sodium puis en une anodisation est beaucoup plus efficace qu'un traitement à l'hydroxyde de sodium seul décrit dans la littérature.

Le traitement à l'hydroxyde de sodium proposé dans l'autre demande de brevet et couplé à une anodisation à 50V est idéal. Il en résulte après une immersion d'une semaine dans du SBF un rapport Ca/P proche de celui de l'os humain et des taux de calcium et de phosphore conséquents.

**Revendications**

1. Procédé de traitement de surface d'un implant en titane bioinerte destiné à être posé en contact avec un os du corps humain, du type consistant à laisser des fluides biologiques, tels que la partie minérale du plasma sanguin humain, déposer naturellement sur l'implant en titane un film de phosphate de calcium, de formule $Ca_5(PO_4)_3(OH)$, sous forme d'hydroxyapatite, en vue de rendre bioactif le titane bioinerte afin de permettre une liaison chimique de l'os avec l'implant, favorisant ainsi l'ostéointégration, le procédé comprenant une opération consistant à immerger le titane dans une solution contenant de l'hydroxyde de sodium NaOH dont la concentration est de l'ordre de 10 moles par litre, ladite immersion entraînant la formation sur le titane d'une couche d'oxydes de titane hydratés ($HTiO_3$-) qui entraîne elle-même l'apparition de groupes hydroxyles TiOH permettant le dépôt dudit film de phosphate de calcium, **caractérisé en ce qu'** il comprend, après l'opération d' immersion de l'implant dans l'hydroxyde de sodium, une opération d'anodisation de l'implant sous une tension donnée permettant une stabilisation de la couche d'oxydes formée par l'opération d'immersion dans l'hydroxyde de sodium.

2. Procédé suivant la revendication 1, **caractérisé en ce que** la tension de l'opération d'anodisation est de l'ordre de 50 à 110 volts.

3. Procédé suivant la revendication 2, **caractérisé en ce que** la tension de l'opération d'anodisation est de l'ordre de 50 à 75 volts.

4. Procédé suivant l'une quelconque des revendications 2 et 3, **caractérisé en ce que** la tension de l'opération d'anodisation est de l'ordre de 50 volts.

5. Procédé suivant l'une quelconque des revendications 1 et 2, **caractérisé en ce que** la tension de l'opération d'anodisation est de l'ordre de 110 volts.

6. Procédé suivant l'une quelconque des revendications 1 à 5, **caractérisé en ce que** chacune des opérations d'immersion dans l'hydroxyde de sodium et d'anodisation est suivie d'une opération de rinçage.

7. Procédé suivant l'une quelconque des revendications 1 à 6, **caractérisé en ce que** l'ensemble des deux opérations d'immersion dans l'hydroxyde de sodium et d'anodisation est suivi d'une opération de séchage.

**8.** Procédé suivant la revendication 7, **caractérisé en ce que** l'opération de séchage est effectuée à 100°C.

**9.** Procédé suivant l'une quelconque des revendications 1 à 8, **caractérisé en ce que** l'ensemble desdites opérations indiquées est suivi d'un nettoyage-lavage standard.

## Patentansprüche

**1.** Verfahren zur Oberflächenbehandlung eines Implantats aus bioinertem Titan, das dazu bestimmt ist, um mit einem Knochen des menschlichen Körpers derart in Kontakt gebracht zu werden, dass biologische Flüssigkeiten, wie beispielsweise dem mineralischen Anteil des menschlichen Blutplasmas, auf dem Titanimplantat auf natürliche Weise einen Calciumphosphatfilm mit der Formel $Ca_5(PO_4)_3(OH)$ in Form von Hydroxyapatit abscheiden können, mit dem Ziel das bioinerte Titan bioaktiv zu machen, eine chemische Bindung zwischen dem Knochen und dem Implantat zu ermöglichen, wodurch Osteointegration gefördert wird,
wobei das Verfahren einen Vorgang zum Eintauchen des Titans in eine Natriumhydroxid NaOH enthaltende Lösung umfasst, deren Konzentration in der Größenordnung von 10 mol pro Liter liegt,
wobei das Eintauchen die Bildung einer Schicht aus hydratisierten Titanoxiden ($HTiO_3^-$) auf dem Titan nach sich zieht, die ihrerseits zum Entstehen von Hydroxylgruppen TiOH führt, die die Abscheidung des Calciumphosphatfilms ermöglichen,
**dadurch gekennzeichnet, dass** es, nach dem Vorgang des Eintauchens des Implantats in das Natriumhydroxid, einen Vorgang der Anodisierung des Implantats unter einer gegebenen Spannung umfasst, der eine Stabilisierung derjenigen Oxidschicht ermöglicht, die durch den Vorgang zum Eintauchen in das Natriumhydroxid gebildet wurde.

**2.** Verfahren nach Anspruch 1, **dadurch gekennzeichnet, dass** die Spannung des Anodisierungsvorgangs in der Größenordnung von 50 bis 110 Volt liegt.

**3.** Verfahren nach Anspruch 2, **dadurch gekennzeichnet, dass** die Spannung des Anodisierungsvorgangs in der Größenordnung von 50 bis 75 Volt liegt.

**4.** Verfahren nach einem der Ansprüche 2 und 3, **dadurch gekennzeichnet, dass** die Spannung des Anodisierungsvorgangs in der Größenordnung von 50 Volt liegt.

**5.** Verfahren nach einem der Ansprüche 1 und 2, **dadurch gekennzeichnet, dass** die Spannung des Anodisierungsvorgangs in der Größenordnung von 110 Volt liegt.

**6.** Verfahren nach einem der Ansprüche 1 bis 5, **dadurch gekennzeichnet, dass** auf jeden der Vorgänge des Eintauchens in das Natriumhydroxid und der Anodisierung ein Spülvorgang folgt.

**7.** Verfahren nach einem der Ansprüche 1 bis 6, **dadurch gekennzeichnet, dass** auf das Ensemble der beiden Vorgänge des Eintauchens in das Natriumhydroxid und der Anodisierung ein Trocknungsvorgang folgt.

**8.** Verfahren nach Anspruch 7, **dadurch gekennzeichnet, dass** der Trocknungsvorgang bei 100 °C durchgeführt wird.

**9.** Verfahren nach einem der Ansprüche 1 bis 8, **dadurch gekennzeichnet, dass** auf das Ensemble der angezeigten Vorgänge eine Standard-Reinigung/Wäsche folgt.

## Claims

**1.** Method for surface processing an implant of bioinert titanium which is intended to be placed in contact with a bone of the human body, of the type involving allowing biological fluids, such as the mineral portion of the human blood plasma, to deposit naturally on the implant of titanium a film of calcium phosphate, having the formula $Ca_5(PO_4)_3(OH)$, in the form of hydroxyapatite, in order to render the bioinert titanium bioactive in order to enable a chemical connection of the bone to the implant, thus promoting osteointegration, the method comprising an operation involving immersing the titanium in a solution containing sodium hydroxide NaOH whose concentration is in the order of 10 moles per litre, the immersion bringing about the formation on the titanium of a layer of hydrated titanium oxides ($HTiO_3^-$) which itself brings about the appearance of hydroxyl groups TiOH which enable the depositing of the film of calcium phosphate, **characterised in that** it comprises, after the operation of immersing the implant in the sodium hydroxide, an operation of anodisation of the implant at a specific voltage which enables stabilisation of the layer of oxides formed by the operation of immersion in the sodium hydroxide.

2. Method according to claim 1, **characterised in that** the voltage of the anodisation operation is in the order of from 50 to 110 volts.

3. Method according to claim 2, **characterised in that** the voltage of the anodisation operation is in the order of from 50 to 75 volts.

4. Method according to either claim 2 or claim 3, **characterised in that** the voltage of the anodisation operation is in the order of 50 volts.

5. Method according to either claim 1 or claim 2, **characterised in that** the voltage of the anodisation operation is in the order of 110 volts.

6. Method according to any one of claims 1 to 5, **characterised in that** each of the operations of immersion in sodium hydroxide and anodisation is followed by a rinsing operation.

7. Method according to any one of claims 1 to 6, **characterised in that** the whole of the two operations of immersion in sodium hydroxide and anodisation is followed by a drying operation.

8. Method according to claim 7, **characterised in that** the drying operation is carried out at 100°C.

9. Method according to any one of claims 1 to 8, **characterised in that** all the operations indicated are followed by a standard cleaning/washing operation.

**FIGURE 1**

**FIGURE 2**

**FIGURE 3**

**FIGURE 4**

**FIGURE 5**

**FIGURE 6**

**FIGURE 7**

**FIGURE 8**

**FIGURE 9**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009044203 A

**Littérature non-brevet citée dans la description**

- **KIENAPFEL H. ; SPREY C. ; WILKE A. ; GRISS P.** Implant fixation by bone ingrowth. *The Journal of Arthroplasty,* 1999, vol. 14 (3
- **KOKUBO T.** Bioactive glass ceramics : properties and applications. *Biomaterials,* 1991, vol. 12, 155-163
- Formation of biologically active bone-like apatite on metals and polymers by a biomimetic process. *Thermochimica Acta 280/281,* 1996, 479-490
- **KOKUBO T. ; TAKADAMA H.** How useful is SBF in predicting in vivo bone bioactivity. *Biomaterials,* 2006, vol. 27, 2907-2915
- **WALÉRIA SILVA DE MEIDEROS ; OLIVEIRA M.V. ; PEREIRA L.C. ; ANDRADE M.C.** Bioactive Porous Titanium : an alternative to surgical implants. *Artificial Organs,* 2008, vol. 32 (4), 277-282
- **KOKUBO.** *Thermochimica Acta,* 1996
- **LENKA JONASOVA ; JONASOVA L. ; MÜLLER F.A. ; HELEBRANT A. ; STRNAD J. ; GREIL P.** Biomimetic apatite formation on chemically treated titanium. *Biomaterials,* 2004, vol. 25, 1187-1194
- Surface nano-functionalization of biomaterials. **LIU X et al.** MATERIALS SCIENCE AND ENGINEERING R: REPORTS. ELSEVIER SEQUOIA S.A, 22 Novembre 2010, vol. 70, 275-302
- Electrodeposition of hydroxyapatite onto nanotubular Ti02 for implant applications. **KAR A et al.** SURFACE AND COATINGS TECHNOLOGY. ELSEVIER, 04 Décembre 2006, vol. 201, 3723-3731
- **DRIESSENS F.C.M. ; BOLTONG M.G. ; DE MAEYER E.A.P. ; WENZ R. ; NIES B. ; PLANELL J.A.** The Ca/P range of nanoapatitic calcium phosphate cements. *Biomaterials,* 2002, vol. 23, 4011-4017